# DEMANDE DE BREVET EUROPEEN

(11) **EP 1 348 392 A1**
(43) Date de publication de la demande: **01.10.2003**
(21) Numéro de dépôt: 03290107.6
(22) Date de dépôt: 15.01.2003
(51) Int. Cl.: A61B 18/14

(54) **Catheter ballon pour electrochirurgie**

(30) Priorité: 27.03.2002 FR 0203850
(71) Demandeur: Integra Neurosciences Implants, 06410 Biot (FR)
(72) Inventeur: Lecuyer, Alain, 06130 Grasse (FR)
(74) Mandataire: Geismar, Thierry

(57) **Abrégé**

L'invention concerne un cathéter-ballon, comprenant un cathéter (1), un ballon (7) comportant une ouverture raccordée de façon étanche à l'extrémité distale du cathéter (1), des moyens de raccordement de l'extrémité proximale du cathéter (1) à des moyens d'injection d'un fluide de gonflage du ballon (7), une électrode (10) solidaire du ballon (7) généralement à l'opposé de son ouverture de raccord avec le cathéter (1), et un fil (14) électriquement conducteur, traversant le cathéter (1) et relié électriquement à son extrémité distale à l'électrode (10) et à son extrémité proximale à des moyens (18) de raccordement à un générateur électrique.

Ledit fil traverse ledit ballon et a son extrémité distale fixée à ladite électrode (10), des moyens (19) étant prévus pour bloquer le coulissement dudit fil à l'intérieur du cathéter.

Application au traitement de l'hydrocéphalie.

## Description

La présente invention concerne un cathéter-ballon, et plus particulièrement un tel cathéter-ballon du type comprenant un cathéter ayant une extrémité distale et une extrémité proximale, un ballon comportant une ouverture raccordée de façon étanche à l'extrémité distale du cathéter, des moyens de raccordement de l'extrémité proximale du cathéter à des moyens d'injection d'un fluide de gonflage du ballon par l'intermédiaire du cathéter, une électrode solidaire du ballon généralement à l'opposé de son ouverture de raccord avec le cathéter, et un fil électriquement conducteur ayant une extrémité distale et une extrémité proximale, traversant le cathéter et relié électriquement à son extrémité distale à l'électrode et à son extrémité proximale à des moyens de raccordement à un générateur électrique.

On connaît en neurochirurgie des opérations au cours desquelles on est appelé à perforer une membrane. Il s'agit par exemple de la perforation du plancher du troisième ventricule cérébral dans le traitement de l'hydrocéphalie.

Ces opérations s'effectuaient traditionnellement en deux temps. Dans un premier temps, la membrane était ouverte, par exemple à l'aide d'un électrocoagulateur, qui était ensuite retiré. On introduisait alors à sa place un cathéter à ballon qui était gonflé afin de dilater l'ouverture.

Il résultait en premier lieu d'une telle procédure une perte de temps, toujours préjudiciable au cours d'une opération. Par ailleurs, il pouvait également se produire un écoulement de fluide céphalo-rachidien lors du retrait de l'électrocoagulateur.

C'est la raison pour laquelle il a été proposé dans le document EP-A-868 883 un cathéter-ballon pour neurochirurgie comprenant un cathéter à l'extrémité duquel était monté un ballon de dilatation, le cathéter étant agencé pour être raccordé à des moyens de gonflage du ballon et comportant en outre à son intérieur un fil d'alimentation électrique pour une électrode formant une pointe d'électrocoagulation fixée au-delà du ballon.

Plus particulièrement, l'extrémité du corps supportait coaxialement un tube métallique à l'extrémité duquel était soudée la pointe d'électrocoagulation et auquel était raccordé électriquement le fil d'alimentation, le ballon étant monté autour du tube métallique dont la paroi était percée pour permettre le gonflement du ballon.

Ainsi le cathéter, ou corps tubulaire, décrit dans ce document antérieur, remplit deux fonctions. D'une part, il sert de conduit pour le fluide de gonflage du ballon. D'autre part, il forme une gaine pour le fil d'alimentation de la pointe d'électrocoagulation.

La perforation de la membrane s'effectue alors en une seule opération. Le cathéter étant mis en place avec la pointe d'électrocoagulation au contact de la membrane à perforer, cette pointe est alimentée et réalise donc une ouverture dans la membrane. Le cathéter est alors légèrement enfoncé de manière à engager partiellement le ballon dans l'ouverture. Ce ballon est enfin gonflé pour dilater l'ouverture après quoi le cathéter est retiré.

L'agencement connu donne généralement satisfaction pour des diamètres relativement importants, par exemple supérieurs à 1mm. Il est en revanche difficile d'obtenir des diamètres inférieurs, compte tenu de la présence du tube métallique de support de la pointe d'électrocoagulation.

La présente invention vise à palier cet inconvénient.

Plus particulièrement, l'invention a pour but de fournir un cathéter-ballon du type rappelé ci-dessus, mais dont le diamètre peut être bien inférieur.

A cet effet, l'invention a pour objet un cathéter-ballon, comprenant un cathéter ayant une extrémité distale et une extrémité proximale, un ballon comportant une ouverture raccordée de façon étanche à l'extrémité distale du cathéter, des moyens de raccordement de l'extrémité proximale du cathéter à des moyens d'injection d'un fluide de gonflage du ballon par l'intermédiaire du cathéter, une électrode solidaire du ballon généralement à l'opposé de son ouverture de raccord avec le cathéter, et un fil électriquement conducteur ayant une extrémité distale et une extrémité proximale, traversant le cathéter et relié électriquement à son extrémité distale à l'électrode et à son extrémité proximale à des moyens de raccordement à un générateur électrique, ledit fil traversant ledit ballon et étant fixé à ladite électrode, des moyens étant prévus pour bloquer le coulissement dudit fil à l'intérieur du cathéter.

Dans un mode de réalisation particulier, ce cathéter-ballon comprend un tronçon de tube par-dessus lequel sont engagées et solidarisées de façon étanche l'extrémité distale du cathéter et l'ouverture du ballon, ledit tronçon de tube ne s'étendant sensiblement pas à l'intérieur du ballon, et ledit fil traversant ledit tronçon de tube en étant solidarisé au tube

Ainsi, le tube ne sert qu'à assurer la liaison étanche entre l'extrémité distale du cathéter et le ballon, et non plus à supporter l'électrode de coagulation. Il ne vient donc pas s'ajouter au diamètre du ballon. L'électrode est directement alimentée électriquement par le fil, qui donne en outre de la rigidité à l'extrémité du cathéter-ballon pour faciliter son introduction.

En outre, le fil étant solidarisé au tube, il ne peut glisser dans le tube et le cathéter lorsqu'une pression est exercée sur l'électrode en vue de son introduction.

Enfin, il n'est plus nécessaire d'effectuer des perçages délicats dans la paroi du tube, comme c'était le cas dans l'art antérieur.

Dans un mode de réalisation particulier, ledit tube est en matériau déformable, et le fil lui est solidarisé par sertissage.

L'électrode peut être sertie sur l'extrémité distale du fil.

L'extrémité distale du cathéter peut être collée sur la surface externe du tronçon de tube.

De même, l'ouverture du ballon peut être collée sur la surface externe du tronçon de tube.

En outre, l'extrémité distale du cathéter et l'ouverture du ballon peuvent être collées entre elles.

Dans un mode de réalisation particulier de l'invention, le ballon est généralement tubulaire et possède une deuxième ouverture opposée à son ouverture de raccord à l'extrémité distale du cathéter, ladite deuxième ouverture étant collée de façon étanche sur ladite électrode.

Egalement dans un mode de réalisation particulier, le ballon possède une restriction de diamètre entre son ouverture de raccordement à l'extrémité distale du cathéter et l'emplacement de solidarisation de l'électrode, de manière à lui éviter de glisser d'un côté ou de l'autre de l'ouverture lors de son gonflement.

On décrira maintenant, à titre d'exemple non limitatif, un mode de réalisation particulier de l'invention, en référence aux dessins schématiques annexés dans lesquels :

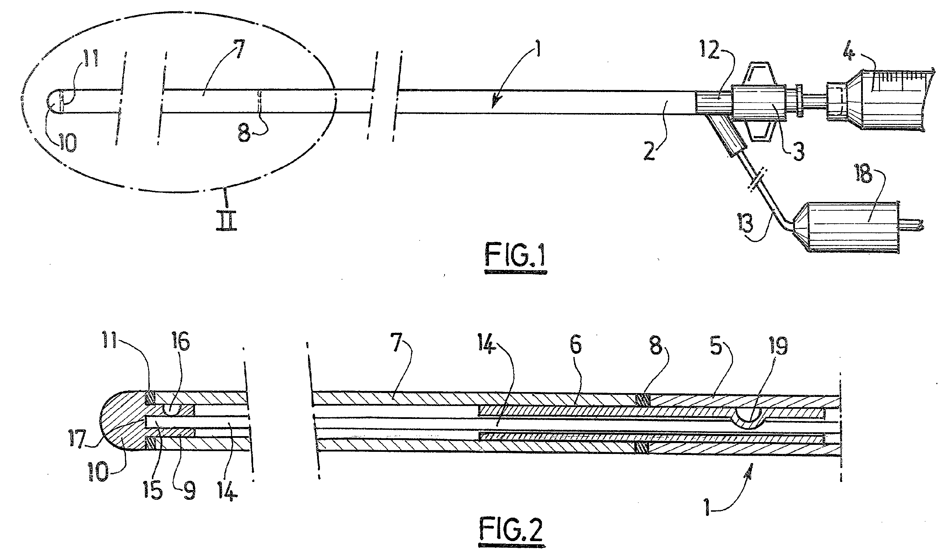

- la figure 1 est une vue de côté d'un cathéter-ballon selon l'invention, dans laquelle le diamètre du cathéter proprement dit a été augmenté pour plus de clarté ; et
- la figure 2 est une vue agrandie en coupe du détail Il de la figure 1.

Ce cathéter-ballon comprend un cathéter 1 tubulaire muni à son extrémité proximale 2 d'un connecteur de gonflage 3. Le connecteur 3 est ici adapté à recevoir l'embout d'une seringue 4.

A son autre extrémité 5, le cathéter 1 est raccordé de façon étanche à un tronçon de tube 6, par exemple en acier inoxydable, par collage ou tout autre moyen convenable, le cathéter et le tronçon de tube étant coaxiaux et une extrémité du tronçon de tube étant engagée dans l'extrémité distale 5 du cathéter 1.

Un ballon en silicone 7 tubulaire a une de ses ouvertures engagée sur l'autre extrémité du tronçon de tube 6. Ce ballon 7 est collé sur le tronçon de tube 6 ou fixé de façon étanche par tout autre moyen. Le tronçon de tube 6 ne pénètre sensiblement pas dans le ballon 7.

Un anneau de colle 8 assure la liaison entre l'ouverture distale du cathéter 1 et l'ouverture du ballon qui est en vis-à-vis.

Le ballon 7 comporte en outre dans sa partie médiane une restriction de diamètre dont la fonction sera exposée ci-après.

L'autre ouverture du ballon 7 est engagée sur une saillie cylindrique 9 d'une électrode 10. Le ballon 7 est collé ou fixé par tout autre moyen convenable sur la saillie 9 et un joint de colle 11 est disposé entre l'ouverture du ballon et l'épaulement de l'électrode. A l'opposé de la saillie 9, l'électrode 10 est sensiblement hémisphérique.

Le cathéter 1 forme à proximité du connecteur 3 un raccord 12 dans lequel pénètre de façon étanche un câble 13 électriquement conducteur. Le fil conducteur 14, relativement rigide, de ce câble 13 se prolonge dans tout le cathéter 1, le tronçon de tube 6 et le ballon 7, et son extrémité distale 15 est finalement sertie en 16 dans un perçage 17 formé dans la saillie 9.

L'extrémité extérieure du câble 13 est reliée à un connecteur standard 18 pour sonde électrocoagulante monopolaire. Ainsi, l'électrode 10 est reliée électriquement au connecteur 18.

Le fil 14 est également serti en 19 dans le tronçon de tube 6 de manière à éviter son coulissement dans le cathéter lors de l'introduction de ce dernier. Le sertissage est toutefois tel qu'il permet la circulation du fluide de gonflage entre le fil 14 et le tronçon de tube 6.

Pour perforer une membrane, on amène en premier lieu l'électrode 10 à son contact, et on l'alimente à partir du connecteur 18. Une ouverture étant ainsi réalisée dans la membrane, l'extrémité du cathéter y est engagée jusqu'à ce que la membrane se trouve au niveau de la restriction de diamètre du ballon 7.

Le ballon 7 est alors gonflé à l'aide de la seringue 4 à partir du connecteur 3 pour dilater l'ouverture de la membrane. Du fait de la restriction de diamètre, le ballon ne peut glisser d'un côté ou de l'autre de la membrane, et reste donc en place.

Lorsque l'ouverture a atteint une dimension suffisante, le ballon 7 est dégonflé et le cathéter peut être retiré.

## Revendications

1. Cathéter-ballon, comprenant un cathéter (1) ayant une extrémité distale (5) et une extrémité proximale (2), un ballon (7) comportant une ouverture raccordée de façon étanche à l'extrémité distale du cathéter (1), des moyens (3) de raccordement de l'extrémité proximale du cathéter (1) à des moyens (4) d'injection d'un fluide de gonflage du ballon (7) par l'intermédiaire du cathéter (1), une électrode (10) solidaire du ballon (7) généralement à l'opposé de son ouverture de raccord avec le cathéter (1), et un fil (14) électriquement conducteur ayant une extrémité distale (15) et une extrémité proximale, traversant le cathéter (1) et relié électriquement à son extrémité distale à l'électrode (10) et à son extrémité proximale à des moyens (18) de raccordement à un générateur électrique, **caractérisé par le fait que** ledit fil traverse ledit ballon et a son extrémité distale fixée à ladite électrode (10), des moyens (19) étant prévus pour bloquer le coulissement dudit fil à l'intérieur du cathéter.

2. Cathéter-ballon selon la revendication 1, comprenant un tronçon de tube (6) par-dessus lequel sont engagées et solidarisées de façon étanche l'extrémité distale du cathéter (1) et l'ouverture du ballon (7), ledit tronçon de tube (6) ne s'étendant sensiblement pas à l'intérieur du ballon (7), et ledit fil (14) traversant ledit tronçon de tube (6) en étant solidarisé au tube

3. Cathéter-ballon selon la revendications 2, dans lequel ledit tronçon de tube (6) est en matériau déformable, et le fil (14) lui est solidarisé par sertissage.

4. Cathéter-ballon selon l'une quelconque des revendications 1 à 3, dans lequel l'électrode (10) est sertie sur l'extrémité distale (15) du fil (14).

5. Cathéter-ballon selon l'une quelconque des revendications 2 à 4, dans lequel l'extrémité distale (5) du cathéter (1) est collée sur la surface externe du tronçon de tube (6).

6. Cathéter-ballon selon l'une quelconque des revendications 2 à 5, dans lequel l'ouverture du ballon (7) est collée sur la surface externe du tronçon de tube (6).

7. Cathéter-ballon selon l'une quelconque des revendications 1 à 6, dans lequel l'extrémité distale (5) du cathéter (1) et l'ouverture du ballon (7) sont collées entre elles.

8. Cathéter-ballon selon l'une quelconque des revendications 1 à 7, dans lequel le ballon (7) est généralement tubulaire et possède une deuxième ouverture opposée à son ouverture de raccord à l'extrémité distale (5) du cathéter (1), ladite deuxième ouverture étant collée de façon étanche sur ladite électrode (10).

9. Cathéter-ballon selon l'une quelconque des revendications 1 à 8, dans lequel le ballon (7) possède une restriction de diamètre entre son ouverture de raccordement à l'extrémité distale (5) du cathéter (1) et l'emplacement (16) de solidarisation de l'électrode (10).
